# EUROPEAN PATENT APPLICATION

(11) **EP 3 011 945 A1**
(43) Date of publication of application: **27.04.2016**
(21) Application number: 14189850.2
(22) Date of filing: 22.10.2014
(51) Int. Cl.: A61J 3/00, B29C 41/36, A61K 9/48

(54) **Apparatus and process for making dosage form articles**

(71) Applicant: Capsugel Belgium NV, 2880 Bornem (BE)
(72) Inventor: Vanquickenborne, Stefaan Jaak, B-2820 Rijmenam (BE); Buydts, Hilde, 2018 Antwerpen (BE); Van Den Abeele, Kris, 8400 Oostende (BE)
(74) Representative: Macchetta, Andrea

(57) **Abstract**

A dipping unit for the manufacture of dip-molded dosage form articles comprising: a reservoir for retaining a liquid composition therein; and a flow imparting unit for imparting a flow to said liquid composition, wherein the flow imparting unit is arranged to impart substantially no flow to the liquid composition, at least in a direction perpendicular to a pin dipping axis, when one or more mold pins are dipped in the liquid composition such that each one or more mold pins is exposed to the same flow when dipped in the liquid composition.

## Description

### FIELD

The present disclosure relates to apparatuses and processes for making dosage form articles, preferably multi-part capsules or two-part hard capsules, typically suitable for the delivery of one or more drugs (or health and nutrition materials) via oral, or other, administration of the same to a subject. More particularly, the dosage form articles are suitable for ingestion by a subject, preferably the subject being selected from humans or animals.

In particular, the present disclosure can be advantageously applied to the manufacture of hard capsules in a dip-molding process.

### BACKGROUND

Receptacle technology, and in particular capsule technology, continues to be subject to development and improvements and so does the manufacture thereof, including processes and equipment. In its basic form, standard containers for pharmaceuticals or other powdered, granular or liquid substances (generally referred to as telescope-type or two-piece capsules) include a tubular-shaped and/or cylindrically-shaped first part, namely a cap part, which is closed on one end and open on the other opposite end. A tightly fitting second part of similar shape, namely the body part, is of smaller diameter than the cap part and is typically telescopically engaged therein to form the overall dosage form or two-piece capsule. Similar capsule technology may be used to generate multi-compartment capsules.

A successful and well established commercial scale production process for this kind of products has been the dip-molding process. Such process typically includes the following broad steps: dipping of mold pins (typically aligned along mold bars) into a solution or dispersion of the desired ingestible material that the capsule is to be made (e.g. gelatin, HPMC and the like), solidification of said material onto the pin, and stripping of the solidified capsule shell from the pin.

Such processes typically utilize a dish (or reservoir) for storing the solution/dispersion in liquid state and for providing a dipping surface into which mold pins are dipped. Such dishes typically comprise an outer dish and an inner dish stored therein and arranged to continuously provide a flow of material from the inner dish into the outer dish to then be recirculated back into the inner dish. Such system is typically referred to as a dish with overflow since continuous flow is ensured by allowing the liquid material to overflow over the side edges of the inner dish and into the outer dish for subsequent recirculation. Examples of this execution are described in US 1,787,777 and US 3,756,759.

Such basic overflow configuration is being utilized still today, although, further improvements have been made in order to address some of the disadvantages that are innate in the general design. An example is described in US 4,997,359, wherein the inner dish is essentially subdivided into smaller units in order to attempt to better control temperature variation between dip molding bars. However, such systems add in complexity and number of parts that not only result in higher cost and difficulty in maintenance but also to the problem of bubble formation and effective reduction in space available for dipping.

There is therefore still a need for a system that overcomes these problems and in particular allows for attaining dosage form articles with low variability in weight whilst limiting the risk of bubble formation and/or enabling the production of more capsules per dipping.

### SUMMARY

A first aspect of the present disclosure relates to a dipping unit for the manufacture of dipmolded dosage form articles comprising: a reservoir for retaining a liquid composition therein; and a flow imparting unit for imparting a flow to said liquid composition, wherein the flow imparting unit is arranged to impart substantially no flow to the liquid composition, at least in a direction perpendicular to a pin dipping axis, when one or more mold pins are dipped in the liquid composition such that each one or more mold pins is exposed to the same flow when dipped in the liquid composition.

A further aspect of the present disclosure relates to an apparatus comprising the dipping unit disclosed herein.

A further aspect of the present disclosure relates to the use of an apparatus and/or dipping unit for the manufacture of dosage form articles.

### BRIEF DESCIPTION OF THE DRAWINGS

Fig. 1 illustrates a section view of a dipping unit according to the state of the art.
Fig. 2 illustrates a section view of a dipping unit according to an embodiment described herein.
Fig. 3 illustrates a section view of a dipping unit according to an embodiment described herein.

### DETAILED DESCRIPTION

By the term "a" and/or "an" when describing a particular element, it is intended "at least one" of that particular element.

By the term "medicament", it is intended a "drug" or the like comprising one or more compounds providing one or more curative benefits to a subject, the terms "medicament" and "drug" may be used interchangeably herein.

By the term "hard shell" or "hard capsule shell", it is intended a shell that is deformable, but which substantially returns to its un-deformed shape upon the removal of a deforming force. Typically such shells comprise less than 25%, preferably less than 20%, more preferably from 0% to 14%, even more preferably from greater than 0% to less than 14%, water by weight.

By the term "substantially no flow", it is intended that the flow is substantially static (e.g. that the velocity of the liquid composition, typically the top surface velocity measured at the uppermost surface of the liquid composition, is not more than 15 mms⁻¹, preferably no more than 10 mms⁻¹, more preferably from 0 mms⁻¹ to 5 mms⁻¹, most preferably about 0 mms⁻¹, at any one position proximal to a pin, preferably each pin. By a position proximal to a pin it is typically intended the velocity measured at a position at a distance of about 0.5d from the center of any one pin, wherein d is the distance between the centers of two consecutive pins. Any method common in the art may be used to measure the velocity, for example, a drop of dye may be placed on the uppermost surface of the liquid composition at the specified position and the time recorded, the distance travelled by the dye is then measured and divided by the recorded time.

By the term "flow", it is generally intended herein the bulk movement of a fluid, typically the liquid composition referred to herein.

By the term "overflow", it is intended a reservoir arranged such to permit flow of a liquid composition out of the reservoir, typically from the apex of the side edges thereof, generally for recirculating the composition back into the reservoir.

Various embodiments will now be described to provide an overall understanding of the principles of the structure, function, manufacture, and use of apparatuses and methods disclosed herein. One or more examples of these embodiments are illustrated in the accompanying figures. Those of ordinary skill in the art will immediately understand that features described or illustrated in connection with one example embodiment can be combined with the features of other example embodiments without generalization from the present disclosure.

### THE DIPPING UNIT

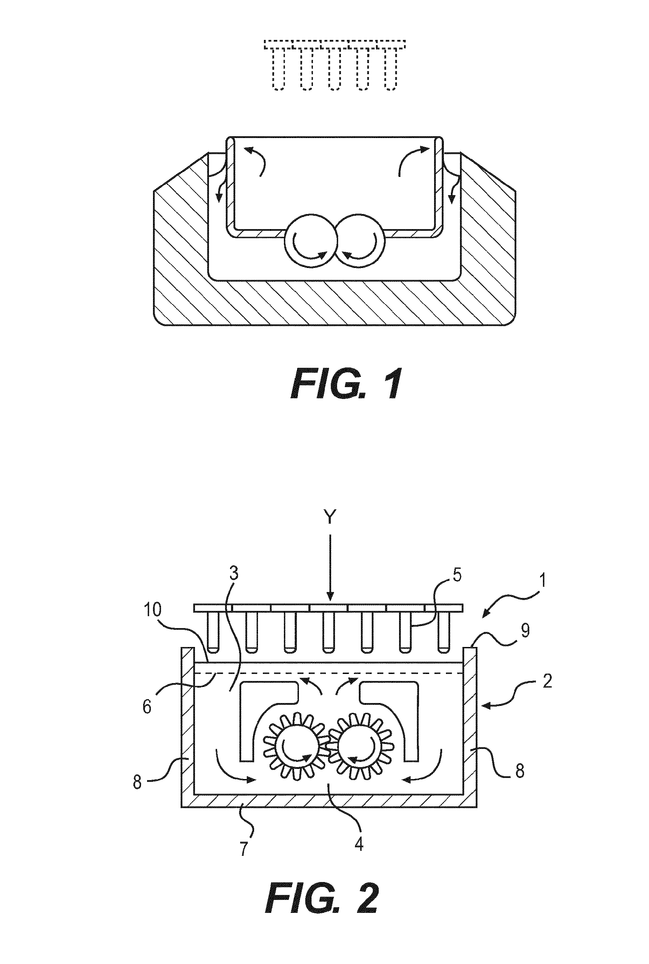

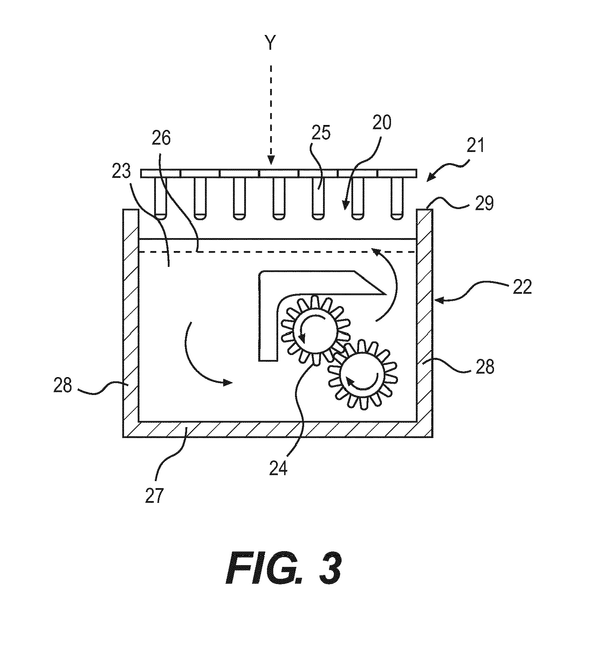

In its basic form (as shown in Fig.2 and Fig. 3), the dipping unit of the present disclosure comprises: (i) a reservoir **2, 22** for retaining a liquid composition **3, 23** therein; and (ii) a flow imparting unit **4, 24** for imparting a flow to said liquid composition **3, 23,** wherein the flow imparting unit **4, 24** is arranged to impart substantially no flow to said liquid composition **3, 23,** at least in a direction perpendicular to a pin dipping axis **Y,** when one or more mold pins **5, 25** are dipped in said liquid composition **3, 23** such that each said one or more mold pins **5, 25** is exposed to substantially the same flow when dipped in said liquid composition **3, 23.** The term "dipped" as used herein generally referring to the state in which the one or more pins are in contact with the liquid composition that is stored in the reservoir (e.g. the bulk composition in the reservoir), at least during immersion and/or emersion of said pins into and/or out of the composition and typically not including the state when the pins are out of the composition stored within the reservoir. It has been found that by ensuring dipping of the pins is carried out in a substantially static composition, a considerable variation in uniformity of the resulting capsules is avoided. Such variation is believed to be otherwise due to the significant flow variations between pins in state of the art overflow arrangements wherein dipping of the pins is carried out in a dynamic composition. Such non-uniformity results in for example substantial variation in the weight of resulting dosage form articles which impacts cost (e.g. unnecessary added material use) as well as quality of the final product (e.g. variability in controlled release in view of non-uniform thickness of the shells). The arrangement herein addresses these issues by enabling uniform coating of the pins through and between pin bars.

In an embodiment, the dipping unit **1,21** comprises a reservoir that is free of overflow, meaning that substantially no flow of composition out of the reservoir, typically from the apex of the side edges thereof is permitted, particularly during dipping of the mold pins. It is desirable to eliminate as much as possible any flow, at least in a direction perpendicular to the dipping axis, since such has been found to be a great driver of non-uniformity in mold pin coating. The nature of the overflow arrangement inevitably results in a substantial flow during dipping, and the elimination of this greatly promotes uniformity.

In an embodiment, the dipping unit may comprise a flow control (not shown) arranged to measure the flow of the liquid composition and to trigger dipping of the pins therein when the substantially no flow condition is detected and/or verified. The flow control may comprise a sensor for measuring the velocity of the liquid composition at the uppermost surface thereof and a control unit, that may comprise a memory device, arranged to compare the velocity measured by the sensor with one or more predetermined values stored in a memory device and arranged to provide a signal to allow the triggering of pin dipping. In this embodiment, when the flow imparting unit stops the flow of liquid composition, the flow control may check the flow conditions and after the substantially no flow of the liquid composition is verified, it permits dipping of the pins, by a trigger thereof. After dipping the pins in the liquid composition (and no pins are in contact with the liquid composition in the reservoir), the flow imparting unit may be activated to impart flow for a given time, and then stopped once again, after which the above explained may be repeated and the flow control may trigger a subsequent dipping of the pins following verification of the no flow condition. The above may be continuously repeated in cycles and synchronized with the dipping of pins into the liquid composition stored in the reservoir.

In an embodiment, the distance between each mold pin and one or more neighboring surfaces, preferably all neighboring surfaces, is substantially the same, typically meaning that the variation in such distance along the same axis is not more than 50%, preferably not more than 40%, preferably not more than 30%, preferably not more than 20%, even more preferably not more than 10%, even more preferably less than 5%, most preferably less than 3%, of the distance between any two consecutive pins along that same axis. By "along the same axis" it is typically intended herein a straight axis that crosses each pin and neighboring surface referred to and is substantially perpendicular to the pin dipping axis **Y.** The neighboring surfaces may be selected from mold pins **5, 25,** one or more surfaces of the reservoir **2, 22,** preferably the side edges thereof, and combinations thereof. An advantage of this arrangement is that flows of the composition (particularly pin-to-pin) is maintained substantially equal also when the one or more pins are immersed in the composition. Without wishing to be bound by theory, it is believed that by ensuring that the distance between each surface is equal, the buoyancy effects due the pins displacing into and out of said composition are equalized throughout the dipping surface such that each pin experiences substantially the same flow conditions throughout the dipping step. Moreover, surface tension variations are further mitigated hence contributing to the overall uniformity of resulting dosage form articles.

In an embodiment, substantially the entire cross sectional surface **6, 26** of the reservoir, typically when in the dipping position, may be useful for dipping the pins **5, 25** therein. By substantially the entire cross sectional surface area it is herein intended at least 80%, preferably at least 85%, more preferably at least 90% of the cross sectional surface area of the reservoir can be useful for dipping pins.

In an embodiment, the dipping unit may further comprise a level adjustment means (not shown) to control the level of liquid composition in the reservoir. The level adjustment means may comprise a buffer tank in fluid communication, preferably liquid communication, with the reservoir, a sensor for measuring the level of liquid composition present in the reservoir, and a control in electrical communication with said sensor. The control may be arranged such that when the level of liquid composition, determined by the sensor, goes below a predetermined value, an amount of liquid composition stored in the buffer tank is allowed to flow into the reservoir up to when the level of the liquid composition in the reservoir, determined by the sensor, reaches said predetermined value. The buffer tank may comprise a mixing means to maintain the liquid composition stored therein in agitation. An advantage of this arrangement is to allow a constant level of liquid composition is kept in the reservoir without the need of an overflow arrangement.

The reservoir typically comprises a base **7, 27;** side edges **8, 28** extending substantially upwardly from said base **7, 27** up to an apex **9, 29;** and an opening **10, 20** defined by the apex of said side edges, the surface of said opening laying on a plane substantially parallel to said base **7, 27,** and wherein said opening **10, 20** delimits the cross-sectional surface area **6, 26** of the reservoir **2, 22.**

The reservoir **2, 22** may be free of recirculation basins proximal to a perimeter thereof, preferably wherein recirculation basins consist of one or more further reservoirs having the purpose of collecting liquid composition **3, 23** released from the reservoir **2, 22,** preferably at the apex of the side edges thereof, for recirculation of said collected liquid composition **3, 23** back into the reservoir **2, 22,** typically when the pins are not dipped.

The flow imparting unit **4, 24** is preferably arranged to impart flow of the liquid composition **3, 23** when the pins **5, 25** are not dipped, preferably in an alternating on-off mode synchronized with the dipping of said pins **5, 25.** This arrangement allows for effective flushing of the reservoir to avoid build-up of cold spots whilst avoiding impacts on uniformity during dipping.

The flow imparting unit **4, 24** may be located within the reservoir **2, 22,** preferably proximal to the base **7, 27** thereof, and may be arranged to impart a substantially uni-directional flow or bidirectional flow. Such arrangement has been found to promote the desired turbulence during flushing to more effectively remove cold spots when the pins are not dipped. In an embodiment, the reservoir may further comprise one or more moveable surfaces preferably located within the reservoir and arranged to alternate between a deployed and un-deployed state typically to further alternate between flushing patterns and/or dipping/no dipping for even more effective cold spot removal and prevention of solidification of the liquid composition at any position of the reservoir, typically at one or more edges thereof.

The flow imparting unit **4, 24** may comprises one or more blades, preferably helical in shape, connected to a drive, said blades being arranged to impart flow of the liquid composition **3, 23** by movement, preferably by rotation, thereof.

In an embodiment, the dipping unit **1, 21** is arranged such that when one or more mold pins **5, 25** are dipped in the liquid composition **3, 23** the flow imparted to said composition by said mold pins, in a direction perpendicular to a mold pin dipping axis Y, is substantially the same between each said pin, typically meaning that the flow at one pin does not vary more than 30%, preferably 20%, more preferably 10%, most preferably 8%, of the flow at the neighboring pin. Typically such flow is that measured at a distance of 0.5d from the center of said pins, wherein d is the distance between the centers of two consecutive pins typically along the same axis.

In a preferred embodiment, the one or more mold pins **5, 25** are shaped so as to correspond to the inverse shape of hard capsule shells, and are preferably substantially identical in shape and size. In addition or alternatively, the mold pins may be substantially tubular in shape and are substantially identical in shape and size. An advantage of such arrangements is to further promote equal flow upon dipping.

The liquid composition described herein typically comprises one or more ingestible materials selected from the group consisting of gelatin, one or more polysaccharides, preferably pullulan; nonionic hydrogels, preferably celluloses such as hydroxypropyl methylcellulose (HPMC), hydroxypropyl methyl cellulose acetate succinate (HPMCAS), hydroxypropyl cellulose (HPCP), cellulose acetate phthalate (CAP); and mixtures thereof, preferably in the form of an aqueous colloidal dispersion of said ingestible material(s).

### THE APPARATUS

The apparatus for making dosage form articles, preferably hard capsules, typically comprises: a dipping unit **1, 21** as described herein; and a mold pin support for retaining and processing one or more mold pins **5, 25.** The mold pin support being arranged to retain and dip said one or more mold pins **5, 25** into the reservoir **2, 22** filled with the liquid composition.

The mold pin support may be arranged to extract the one or more mold pins **5, 25** from the reservoir **2, 22** following dipping, and to rotate said pins 180° about a rotation axis perpendicular to the dipping axis **Y** prior to further processing of the one or more mold pins **5, 25** coated with the liquid composition **3, 23.**

### THE DOSAGE FORM ARTICLES

The dosage form articles herein may be made of, or consist of, an ingestible material comprising materials selected from the group consisting of gelatin, one or more polysaccharides, preferably pullulan; nonionic hydrogels, preferably cellulose such as hydroxypropyl methylcellulose (HPMC); and mixtures thereof. Most preferred materials being gelatin and/or hydroxypropyl methylcellulose (HPMC). Dosage form articles herein may be non-injection molded, and preferably made via a dip molding process. The latter ensures high production speeds and cost effectiveness. Other materials may also be used, as will be recognized by one skilled in the art, including cellulose ethers, such as starches (e.g. waxy maize starch, tapioca dextrin, and derivatives thereof), carrageenan, and polymers or copolymers of (meth)acrylic acids and derivatives thereof.

Typically, the receptacles are in the form of two-piece hard capsules comprising cap and body parts that may be substantially tubular in shape and each comprise a single opening.

### MEDICAMENT

Drugs (i.e. medicaments) suitable for use in the dosage form articles described herein may take any form and be for any treatment of a human or animal subject. This includes not only pharmaceutical compounds but also dietary supplements such as vitamins, minerals and the like.

The drug may be in a state selected from solid or liquid, preferably solid, at room temperature and atmospheric pressure, and comprises one or more active compounds.

Suitable compounds for delivery according to the disclosure include, but are not limited to, particulate, powder, waxy, liquid, and/or pellet forms of the following:
a) pharmaceuticals (also called pharmaceutical actives) such as betamethasone, thioctic acid, sotalol, salbutamol, norfenefrine, silymahn, dihydroergotamine, buflomedil, etofibrate, indomethacin, oxazepam, acetyldigitoxins, piroxicam, halopehdol, isosorbide mononitrate, amithptyline, diclofenac, nifedipine, verapamil, pyritinol, nitrendipine, doxy- cycline, bromhexine, methylprednisolone, clonidine, fenofibrate, allopurinol, pirenzepine, levothyroxine, tamoxifen, metildigoxin, o-(B-hydroxyethyl)-rutoside, propicillin, aciclovirmononitrate, paracetamolol, naftidrofuryl, pentoxifylline, propafenone, acebutolol, 1- thyroxin, tramadol, bromocriptine, loperamide, ketofinen, fenoterol, ca-dobesilate, propranolol, minocycline, nicergoline, ambroxol, metoprolol, B-sitosterin, enalaprilhydro- genmaleate, bezafibrate, isosorbide dinitrate, gallopamil, xantinolnicotinate, digitoxin, flunitrazepam, bencyclane, depanthenol, pindolol, lorazepam, diltiazem, piracetam, phenoxymethylpenicillin, furosemide, bromazepam, flunarizine, erythromycin, metoclo- pramide, acemetacin, ranitidine, biperiden, metamizol, doxepin, dipotassiumchloraze- pat, tetrazepam, estramustinephosphate, terbutaline, captopril, maprotiline, prazosin, atenolol, glibenclamid, cefaclor, etilefrin, cimetidine, theophylline, hydromorphone, ibu- profen, primidone, clobazam, oxaceprol, medroxyprogesterone, flecainide, Mg- pyhdoxal-5-phosphateglutaminate, hymechromone, etofyllineclofibrate, vincamine, cin- narizine, diazepam, ketoprofen, flupentixol, molsidomine, glibornuhde, dimethindene, melperone, soquinolol, dihydrocodeine, clomethiazole, clemastine, glisoxepid, kallidino- genase, oxyfedhne, baclofen, carboxymethylcystsin, thioredoxin, betahistine, 1-tryptophan, myrtol, bromelain, prenylamine, salazosulfapyridine, astemizole, sulpiride, benzerazid, dibenzepin, acetylsalicylic acid, miconazole, nystatin, ketoconazole, sodium picosulfate, colestyramate, gemfibrozil, rifampin, fluocortolone, mexiletine, amoxicillin, terfenadine, mucopolysaccharidpolysulfuric acid, triazolam, mianserin, tiaprofensaure, ameziniummethylsulfate, mefloquine, probucol, quinidine, carbamazepine, Mg-1- aspartate, penbutolol, piretanide, amitriptyline, caproteron, sodium valproinate, me- beverine, bisacodyl, 5-amino-salicyclic acid, dihydralazine, magaldrate, phenprocou- mon, amantadine, naproxen, carteolol, famotidine, methyldopa, auranofine, estriol, nadolol, levomepromazine, doxorubicin, medofenoxat, azathioprine, flutamide, norfloxacin, fendiline, prajmaliumbitartrate, aescin acromycin, anipamil, benzocaine, [beta]- carotene, cloramphenicol, chlorodiazepoxid, chlormadinoneacetate, chlorothiazide, cinnarizine, clonazepam, codeine, dexamethasone, dicumarol, digoxin, drotaverine, gramicidine, griseofulvin, hexobarbital hydrochlorothiazide, hydrocortisone, hydroflumethiazide, ketoprofen, lonetil, medazepam, mefruside, methandrostenolone, sulfaperine, nalidixic acid, nitrazepam, nitrofurantoin, estradiol, papaverine, phenacetin, phenobarbi- tal, phenylbutazone, phenytoin, prednisone, reserpine, spironolactine, streptomycin, sulfamethizole, sulfamethazine, sulfamethoxoazole, sulfamethoxydiazinon, sulfathiazole, sulfisoxazole, testosterone, tolazamide, tolbutamide, trimethoprim, tyrothricin, antacids, reflux suppressants, antiflatulents, antidopaminergics, proton pump inhibitors, H2- receptor antagonists, cytoprotectants, prostaglandin analogues, laxatives, antispasmodics, antidiarrhoeals, bile acid sequestrants, opioids, beta-receptor blockers, calcium channel blockers, diuretics, cardiac glycosides, antiarrhythmics, nitrates, antianginals, vasoconstrictors, vasodilators, ACE inhibitors, angiotensin receptor blockers, alpha blockers, anticoagulants, heparin, antiplatelet drugs, fibrinolytic, anti-hemophilic factor, haemostatic drugs, hypolipidaemic agents, statins, hypnotics, anaesthetics, antipsychotics, antidepressants (including tricyclic antidepressants, monoamine oxidase inhibitors, lithium salts, selective serotonin reuptake inhibitors), anti-emetics, anticonvulsants, an- tiepileptics, anxiolytics, barbiturates, movement disorder drugs, stimulants (including amphetamines), benzodiazepine, cyclopyrrolone, dopamine antagonists, antihistamines, cholinergics, anticholinergics, emetics, cannabinoids, 5-HT antagonists, analgesics, muscle relaxants, antibiotics, sulfa drugs, aminoglycosides, fluoroquinolones, bronchodilators, NSAIDs, antiallergy drugs, antitussives, mucolytics, decongestants, corticosteroids, beta-receptor antagonists, anticholinergics, steroids, androgens, antian- drogens, gonadotropin, corticosteroids, growth hormones, insulin, antidiabetic drugs (including sulfonylurea, biguanide/metformin, and thiazolidinedione), thyroid hormones, antithyroid drugs, calcitonin, diphosponate, vasopressin analogs, contraceptives, follicle stimulating hormone, luteinising hormone, gonadotropin release inhibitor, progestogen, dopamine agonists, oestrogen, prostaglandin, gonadorelin, clomiphene, tamoxifen, di- ethylsti I bestrol , antimalarials, anthelmintics, amoebicides, antivirals, antiprotozoals, vaccines, immunoglobulin, immunosuppressants, interferon, monoclonal antibodies, and mixtures thereof;
b) vitamins, e.g., fat-soluble vitamins such as vitamins A, D, E, and K, and water soluble vitamins such as vitamin C, biotin, folate, niacin, pantothenic acid, riboflavin, thiamin, vitamin B6, vitamin B12, and mixtures thereof;
c) minerals, such as calcium, chromium, copper, fluoride, iodine, iron, magnesium, manganese, molybdenum, phosphorus, potassium, selenium, sodium (including sodium chloride), zinc, and mixtures thereof;
d) dietary supplements such as herbs or other botanicals, amino acids, and substances such as enzymes, organ tissues, glandulars, and metabolites, as well as concentrates, metabolites, constituents, extracts of dietary ingredients, and mixtures thereof;
e) homoeopathic ingredients such as those listed in the Homeopathic Pharmacopoeia of the United States Revision Service (HPRS) , and mixtures thereof. It must be recognized, of course, that the HPRS is periodically updated and that the present invention includes homeopathic ingredients that may be added to the HPRS; and mixtures in any combination of the foregoing.

### THE PROCESS

The process for making dip-molded dosage form articles, preferably two-part hard capsules, comprises the steps of: providing a dipping unit **1, 21** as described herein; filling said dipping unit **1, 21** with a liquid composition **3, 23** having the material characteristics of the desired dosage form articles; providing an apparatus as described herein comprising said dipping unit **1, 21** filled with said composition **3, 23;** and dipping the one or more mold pins **5, 25** in said liquid composition **3, 23.**

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm" (i.e. every value in a practical range close to 40 mm).

## Claims

1. A dipping unit (1, 21) for the manufacture of dip-molded dosage form articles comprising:
(i) a reservoir (2, 22) for retaining a liquid composition (3, 23) therein; and
(ii) a flow imparting unit (4, 24) for imparting a flow to said liquid composition (3, 23),
**characterized in that** the flow imparting unit (4, 24) is arranged to impart substantially no flow, to said liquid composition (3, 23), at least in a direction perpendicular to a pin dipping axis (Y), when one or more mold pins (5, 25) are dipped in said liquid composition (3, 23) such that each said one or more mold pins (5, 25) is exposed to the same flow when dipped in said liquid composition (3, 23).

2. A dipping unit (1, 21) according to claim 1 wherein the reservoir is free of overflow.

3. A dipping unit (1, 21) according to any of the preceding claims wherein the distance between each mold pin and one or more neighboring surfaces along the same axis is substantially the same, preferably wherein the neighboring surfaces are selected from mold pins (5, 25), one or more surfaces of the reservoir (2,22) and combinations thereof.

4. A dipping unit (1, 21) according to any of the preceding claims wherein the dip-molded dosage form articles comprise, preferably consist of, hard capsule parts.

5. A dipping unit (1, 21) according to any of the preceding claims wherein the reservoir comprises a base (7, 27); side edges (8, 28) extending substantially upwardly from said base (7, 27) up to an apex (9, 29); and an opening (10, 20) defined by the apex of said side edges, the surface of said opening laying on a plane substantially parallel to said base (7, 27), and wherein said opening (10, 20) delimits the cross-sectional surface area (6, 26) of the reservoir (2, 22).

6. A dipping unit (1, 21) according to any of the preceding claims wherein the reservoir (2, 22) is free of recirculation basins proximal to a perimeter thereof, preferably wherein recirculation basins consist of one or more further reservoirs having the purpose of collecting liquid composition (3, 23) released from the reservoir (2,22), preferably at the apex of the side edges thereof, for recirculation of said collected liquid composition (3, 23) back into the reservoir (2, 22) typically when the pins are not dipped.

7. A dipping unit (1, 21) according to any of the preceding claims wherein the flow imparting unit (4, 24) is arranged to impart flow of the liquid composition (3, 23) when the pins (5, 25) are not dipped, preferably in an alternating on-off mode synchronized with the dipping of said pins (5, 25).

8. A dipping unit (1, 21) according to any of the preceding claims wherein the flow imparting unit (4, 24) is located within the reservoir (2, 22), preferably proximal to the base (7, 27) thereof, and is arranged to impart a substantially uni-directional flow or bi-directional flow.

9. A dipping unit (1, 21) according to any of the preceding claims arranged such that when one or more mold pins (5, 25) are dipped in the liquid composition (3, 23) the flow imparted to said composition by said mold pins, in a direction perpendicular to a mold pin dipping axis (Y), is substantially the same between each said pin.

10. A dipping unit (1, 21) according to any of the preceding claims wherein the one or more mold pins (5, 25) are shaped so as to correspond to the inverse shape of hard capsule shells, and are preferably substantially identical in shape and size.

11. A dipping unit (1, 21) according to any of the preceding claims wherein the liquid composition comprises one or more ingestible materials selected from the group consisting of gelatin, one or more polysaccharides, preferably pullulan; nonionic hydrogels, preferably celluloses such as hydroxypropyl methylcellulose (HPMC), hydroxypropyl methyl cellulose acetate succinate (HPMCAS), hydroxypropyl cellulose (HPCP), cellulose acetate phthalate (CAP); and mixtures thereof, preferably in the form of an aqueous colloidal dispersion of said ingestible material(s).

12. An apparatus comprising:
(i) a dipping unit (1, 21) according to any of the preceding claims; and
(ii) a mold pin support for retaining and processing one or more mold pins (5, 25),
wherein the mold pin support is arranged to retain and dip said one or more mold pins (5, 25) into the reservoir (2, 22) filled with the liquid composition.

13. An apparatus according to claim 12 wherein the mold pin support is arranged to extract the one or more mold pins (5, 25) from the reservoir (2, 22) following dipping, and to rotate said pins 180° about a rotation axis perpendicular to the dipping axis (Y) prior to further processing of the one or more mold pins (5, 25) coated with the liquid composition (3, 23).

14. An apparatus according to claims 12 to 13 wherein the one or more mold pins (5, 25) are substantially tubular in shape and are substantially identical in shape and size.

15. A process for making dip-molded dosage form articles, preferably two-part hard capsules, comprising the steps of:
(i) providing a dipping unit (1, 21) according to claims 1 to 11;
(ii) filling said dipping unit (1, 21) with a liquid composition (3, 23) having the material characteristics of the desired dosage form articles;
(iii) providing said dipping unit (1, 21) filled with said composition (3, 23) in an apparatus according to claims 12 to 14; and
(iv) dipping the one or more mold pins (5, 25) in said liquid composition (3, 23).
